# EUROPEAN PATENT APPLICATION

(11) **EP 2 618 155 A1**
(43) Date of publication of application: **24.07.2013**
(21) Application number: 11824745.1
(22) Date of filing: 08.09.2011
(51) Int. Cl.: G01N 33/66

(54) **BIOLOGICAL SAMPLE MEASUREMENT SYSTEM**

(30) Priority: 16.09.2010 JP 2010207497
(71) Applicant: Panasonic Corporation, Osaka 571-8501 (JP)
(72) Inventor: MATSUMURA, Keisuke, Osaka-shi, Osaka 540-6207 (JP)
(74) Representative: Eisenführ, Speiser & Partner
(86) International application number: PCT/JP2011/005047
(87) International publication number: WO 2012/035726

(57) **Abstract**

The present invention comprises a biological sample data management device (1) and biological sample measuring devices (2, 3, 4) connected to the biological sample data management device (1). At the biological sample measuring device (2), the brightness of a display section (7) and/or the volume of an operation sound generator (12) is set as a first setting, this first setting is transmitted through the biological sample data management device (1) to the biological sample measuring devices (3, 4), and the brightness of the display section (7) or the volume of the operation sound generator (12) of these biological sample measuring devices (3, 4) is set as a first setting.

## Description

### TECHNICAL FIELD

The present invention relates to a biological sample measurement system that includes biological sample measuring devices for measuring biological samples, such as blood glucose levels or lactic acid levels.

### BACKGROUND ART

Conventional biological sample measuring devices are widely used in hospitals and other such medical institutions, for example. In a hospital, biological samples need to be measured for many patients at regular times, so a plurality of biological sample measuring devices are installed in a nurse center.

The nurses carry the biological sample measuring devices to hospital rooms, where they measure blood glucose levels, for example. They then return to the nurse center and transmit the measurement values from the biological sample measuring devices to a biological sample data management device (see Patent Literature 1, for example).

### CITATION LIST

### PATENT LITERATURE

Patent Literature 1: Japanese Laid-Open Patent Application 2002-2913

### SUMMARY

However, the following problems are encountered with the conventional biological sa mple measuring devices mentioned above.

### TECHNICAL PROBLEM

When a patient's blood glucose level is measured in a hospital, it must be measured at least eight times, such as at 6:30 when the patient wakes up, at 8:00 before breakfast, at 9:30 after breakfast, at 11:30 before lunch, at 1:00 after lunch, at 5:30 before dinner, at 7:00 after d inner, and at 8:30 before going to bed.

Thus, since the blood glucose level is measured eight times a day, when it is measured at one of the above times, such as after the patient wakes up, consideration must be afforded to other patients whose blood glucose level is not measured.

More specifically, the biological sample measuring devices used to measure blood glu cose level comprise a measurement section, a first controller that is connected to the measure ment section, a first display section that is connected to the first controller, a first recorder, a f irst input section, an operation sound generator, an operation setting memory, and a first data transceiver. A beep, for example, is emitted from the operation sound generator every time a measurement operation is conducted. This beeping sound may awaken patients other than the one being measured, and this can be unpleasant.

Therefore, particularly when a measurement is made right after a patient wakes up, co nsideration must be given to lower the volume of the beeps or other sounds emitted from the operation sound generator.

When measurements are subsequently made before or after breakfast, by then all the o ther patients will have awakened, and the area around the person being measured will be relat ively noisy. Therefore, unlike above, the volume of the beeps needs to be increased so that th e user can confirm proper operation of the device.

Then, when measurements are made before bedtime, the beeps emitted from the opera tion sound generator need to be lowered, and the brightness of the first display section reduce d, so as not to awaken the other patients who may already have gone to sleep.

In other words, with a conventional biological sample measuring device, the setting v alues for the biological sample measuring device have to be changed a number of times as me asurements are made throughout the day. Furthermore, in a hospital or other such medical ins titution, since a plurality of biological sample measuring devices are used, changing these sett ing values becomes quite cumbersome and makes the device less convenient to use.

In view of this, it is an object of the present invention to provide a biological sample measurement system with which the setting values of a biological sample measuring device c an be efficiently changed even when a plurality of biological sample measuring devices are in use in a hospital or other such medical institution.

### SOLUTION TO PROBLEM

To achieve the stated object, the present invention comprises first and second biologic al sample measuring devices that measure biological samples. The first and second biologica 1 sample measuring devices each have a measurement section, a controller, a display section, a recorder, an input section, and a data transceiver. The measurement section measures biolo gical samples. The controller is connected to the measurement section. The display section i s connected to the controller and displays setting values and measurement results at the meas urement section. The recorder stores the setting values and the measurement results. The inp ut section is used for inputting the setting values. The data transceiver is used for sending an d receiving information related to the setting values. At the first biological sample measuring device, the controller transmits information related to setting values that have been updated t hrough the input section to the second biological sample measuring device via the data transc eiver. At the second biological sample measuring device, the controller updates the setting va lues on the basis of information related to setting values transmitted from the first biological s ample measuring device.

### ADVANTAGEOUS EFFECTS

With the present invention, if the first biological sample measuring device is set to a first setting, this first setting will be transmitted to the second biological sample measuring device, and the second biological sample measuring device will be automatically set to the first setting. As a result, even when a plurality of biological sample measuring devices are used with the same setting values, each biological sample measuring device does not have to be individually operated and set, and this makes the devices more convenient for the users.

### BRIEF DESCRIPTION OF DRAWINGS

FIG. 1 is a diagram of the configuration of the biological sample measurement system including biological sample measuring devices pertaining to an embodiment of the present invention;
FIG. 2 is an electrical block diagram of the biological sample measurement system in FIG. 1;
FIG. 3 is an operational flowchart of the biological sample measurement system in FIG. 1;
FIG. 4 is an operational flowchart of the biological sample measurement system in FIG. 1;
FIG. 5 is a diagram of the configuration of the biological sample measurement system including biological sample measuring devices pertaining to another embodiment of the present invention;
FIG. 6 is an electrical block diagram of the biological sample measurement system in FIG. 5;
FIG. 7 is an operational flowchart of the biological sample measurement system in FIG. 5;
FIG. 8 is a diagram of the configuration of the biological sample measurement system including biological sample measuring devices pertaining to yet another embodiment of the present invention;
FIG. 9 is an electrical block diagram of the biological sample measurement system in FIG. 8;
FIG. 10 is an operational flowchart of the biological sample measurement system in FIG. 8;
FIG. 11 is an operational flowchart of the biological sample measurement system in FIG. 8;
FIG. 12 is an electrical block diagram of the biological sample measurement system in FIG. 8;
FIG. 13 is an operational flowchart of the biological sample measurement device in FIG. 8;
FIG. 14 is an operational flowchart of the biological sample measurement device in FIG. 8;
FIG. 15 is a diagram of the configuration of the biological sample measurement system including biological sample measuring devices pertaining to yet another embodiment of the present invention;
FIG. 16 is an electrical block diagram of the biological sample measurement system in FIG. 15; and
FIG. 17 is an operational flowchart of the biological sample measurement device in FIG. 15.

### DESCRIPTION OF EMBODIMENTS

The biological sample measurement system pertaining to an embodiment of the present invention will be described in detail through reference to the drawings.

### Embodiment 1

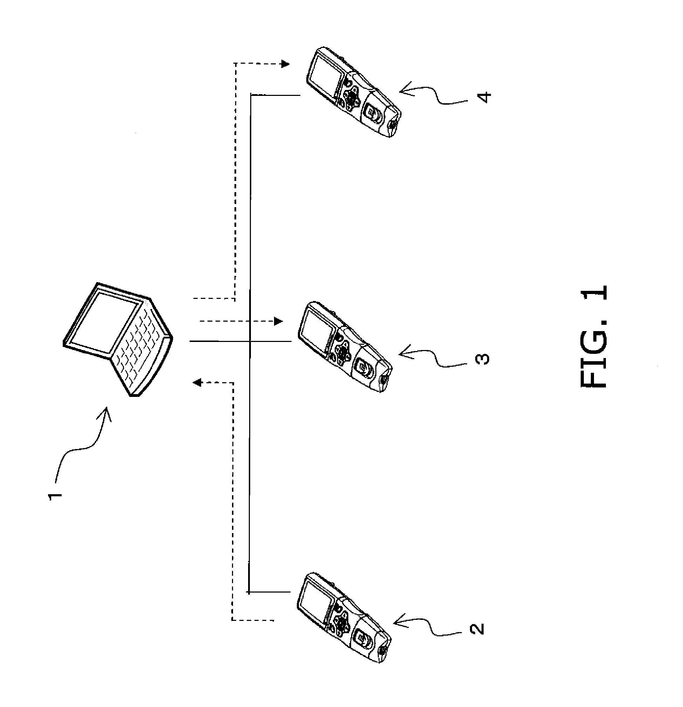

As shown in FIG. 1, the biological sample measurement system in this embodiment comprises a biological sample data management device 1 installed in the nurse center of a hospital, and biological sample measuring devices 2, 3, and 4 that are electrically connected to the biological sample data management device 1.

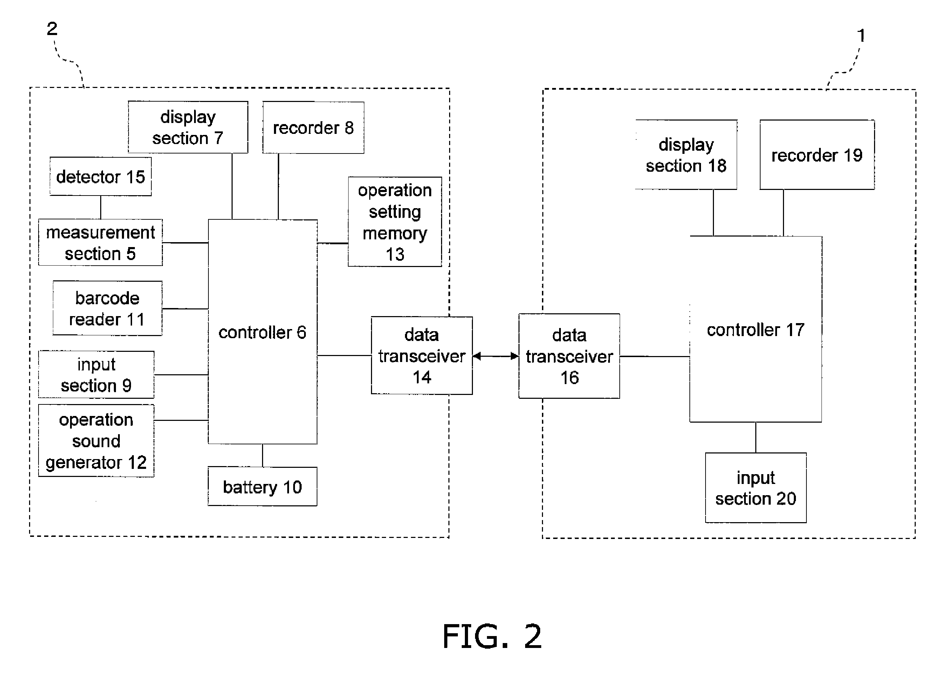

The biological sample measuring devices 2, 3, and 4 are connected to the biological sample data management device 1 as shown in FIGS. 1 and 2.

The biological sample measuring devices 2, 3, and 4 all have basically the same configuration, so FIG. 2 will be described by using the configuration of the biological sample measuring device 2 as an example.

The biological sample measuring device 2 has a measurement section 5 that measures blood glucose level, a controller 6 that is connected to the measurement section 5, a display section 7 that is connected to the controller 6, a recorder 8, an input section 9, a battery 10, a barcode reader 11, an operation sound generator 12, an operation setting memory (recorder) 13, and a data transceiver 14.

The measurement section 5 is connected to a detector 15 that detects when a blood glucose sensor (not shown) has been mounted to the biological sample measuring device 2.

In FIG. 2, the recorder 8 and the operation setting memory 13 are provided separately, with the recorder 8 mainly holding blood glucose levels and other such measurement-related data, and the operation setting memory 13 holding setting values, determination values, various parameters, and other such data. However, the recorder 8 and the operation setting memory 13 do not necessarily have to be provided separately.

That is, a data area for operation setting values may be left in the recorder 8, and the operation setting memory 13 may be collected in the recorder 8.

The input section 9 corresponds to control buttons (such as a power button (start button), an enter button, a menu button, a cross key or ten-key (number keys) for selecting and setting, and so forth) provided on the outer surface of the biological sample measuring devices 2, 3, and 4, and is used to change and refer to setting values, refer to measurement values, switch the mode, and so on.

When the biological sample measuring device 2 is used to find a blood glucose level, first, as is commonly known, the input section 9 is used to turn on the power or select the measurement mode, and the nurse's ID, the patient's ID, and the ID of the blood glucose sensor are read by the barcode reader 11.

These operations are performed because the biological sample measuring devices 2, 3, and 4 in this embodiment are measurement devices intended for use in a medical institution. More specifically, the biological sample measuring devices 2, 3, and 4 in this embodiment will be used on a plurality of patients, and will be used by a plurality of users (such as nurses), so these operations are essential in terms of the accurate management of measurement data. This concludes measurement preparations.

Next, a blood glucose sensor is mounted to the sensor mounting portion (not shown) of the biological sample measuring device 2, a finger of the patient is punctured and blood squeezed out, and this blood is deposited on the blood glucose sensor. The blood glucose level is then measured by the measurement section 5 and displayed on the display section 7. This measurement value and the above-mentioned ID numbers are associated with each other and recorded to the recorder 8.

The detector 15 provided to the sensor mounting portion at this point detects whether or not the blood glucose sensor has been properly mounted (such as determining whether or not the blood glucose sensor is backwards, or whether it is the wrong type of blood glucose sensor). After measurement, information about the time of measurement is also recorded to the recorder 8 in association with the measurement value, patient ID, nurse ID, and so forth.

This operating mode is executed according to setting values recorded to the operation setting memory 13. In this embodiment, the setting values of the biological sample measuring device 2 are set and changed by input through the input section 9.

The operation setting memory 13 is constituted by an EEPROM (electrically erasable programmable read-only memory), which is a kind of nonvolatile memory, so that these setting values can be set and later changed.

In FIG. 2, the operation setting memory 13 is fixed in the interior of the biological sample measuring device 2, but an interchangeable memory (such as an SD memory) or the like can be used instead. Similarly, an interchangeable memory (such as an SD memory) can be used for the recorder 8.

Although this will be described in detail at a later point, the input and changing of the setting values in the operation setting memory 13 of the biological sample measuring devices 3 and 4 are accomplished by transmitting the setting values set in the operation setting memory 13 of the biological sample measuring device 2 through the biological sample data management device 1 to the biological sample measuring devices 3 and 4, and writing them to the operation setting memories 13 of the biological sample measuring devices 3 and 4.

As a result, the biological sample measuring devices 2, 3, and 4 are set to and operate at the same setting values.

As shown in FIG. 2, the biological sample data management device 1 has a data transceiver 16 that sends and receives data wirelessly (or through wires) to and from the data transceiver 14 of the biological sample measuring device 2, a controller 17 that is connected to the data transceiver 16, and a display section 18, a recorder 19, and an input section 20 that are connected to the controller 17.

That is, the blood glucose measurement value, which has been associated with the various ID information transmitted from the recorder 8 of the biological sample measuring device 2 to the biological sample data management device through the data transceivers 14 and 16, is displayed on the display section 18 and recorded to the recorder 19.

As shown in FIG. 1, the biological sample measuring devices 3 and 4 are also linked to the biological sample data management device 1. Therefore, blood glucose measurement values associated with the various ID information obtained by the biological sample measuring devices 3 and 4 is displayed on the display section 18 and recorded to the recorder 19.

The input section 20 is operated when data is exchanged between the biological sample data management device 1 and the biological sample measuring devices 2, 3, and 4. Changing the Setting values of the Biological Sample Measuring Devices 2, 3, and 4

The biological sample measuring devices 2, 3, and 4 included in the biological sample measurement system of this embodiment are used to measure patients' blood glucose levels in a hospital. The blood glucose level is measured, for example, eight times a day in the case of most patients.

More specifically, the blood glucose level must be measured at least eight times, namely, at 6:30 when the patient wakes up, at 8:00 before breakfast, at 9:30 after breakfast, at 11:30 before lunch, at 1:00 after lunch, at 5:30 before dinner, at 7:00 after dinner, and at 8:30 before going to bed.

Thus, since the blood glucose level is measured eight times a day, when it is measured right after the patient wakes up, for example, consideration must be afforded to other patients whose blood glucose level is not measured.

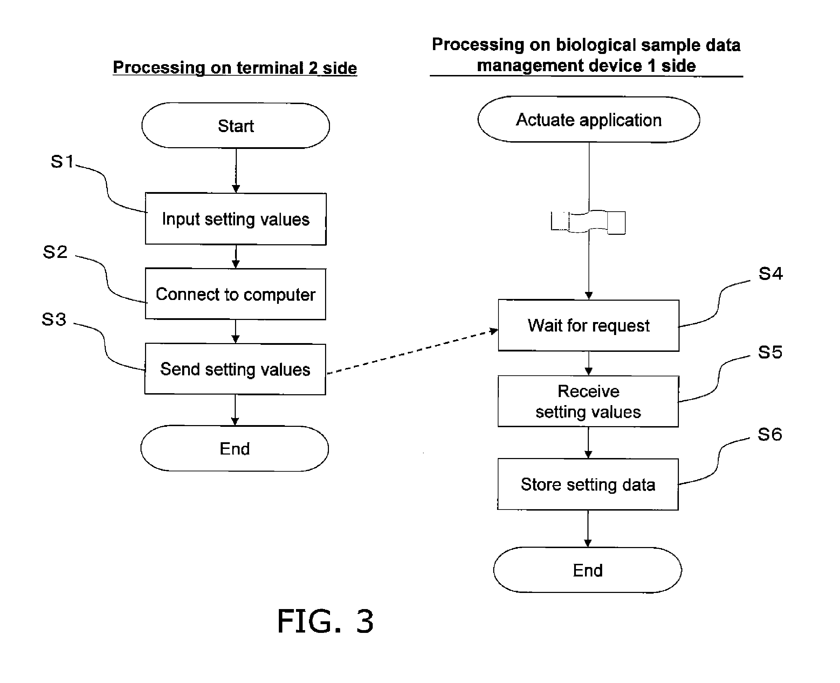

The procedure entailed in changing the setting values of the biological sample measuring devices 2, 3, and 4 in the biological sample measurement system of this embodiment will be described through reference to the flowchart in FIG. 3.

In the following description, an example will be given in which setting changes to the biological sample measuring device 2 (first biological sample measuring device) are inputted manually, and setting changes to the other biological sample measuring devices (second biological sample measuring devices) 3 and 4 are carried out automatically.

### Right after Waking

When a blood glucose level is to be measured, a beep is emitted from the operation sound generator 12 to notify the user of the progress in the measurement operation of the biological sample measuring device 2, etc. Thus, the setting of the biological sample measuring device 2, etc., being used must be changed so that this beeping sound will not awaken patients other than the one being measured, or cause any discomfort on the part of the other patients.

In view of this, with the biological sample measuring devices 2, 3, and 4 in this embodiment, when a measurement is to be made right after a patient wakes up, the setting is changed so that the volume of the beep emitted from the operation sound generator 12 is reduced.

That is, the setting of the biological sample measuring device 2 is changed by the nurse of other user at the nurse station before measurement right after the patient wakes up, or more specifically, after the last measurement of the previous day, so as to lower the volume of the beep coming out of the operation sound generator 12 by changing the setting values recorded to the operation setting memory 13 via the input section 9 of the biological sample measuring device 2 (S1).

Next, the biological sample measuring device 2 whose setting values have been changed is connected to the biological sample data management device 1 (S2).

Next, the input section 9 of the biological sample measuring device 2 is operated to send the changed setting values to the biological sample data management device 1 (S3).

At this point, the biological sample data management device 1 enters a state of awaiting a request (S4).

Thus, the changed setting values are received from the biological sample measuring device 2 (S5), and then these setting values are stored in the recorder 19 of the biological sample data management device 1 (S6).

Next, the power is turned on to the biological sample measuring devices 3 and 4 that have returned to the nurse station (S7).

Next, the passwords for the biological sample measuring devices 3 and 4 are inputted (S8).

Next, a setting reception request is sent to the biological sample data management device 1 (S9).

At this point the biological sample data management device 1 enters a state of awaiting a request (S 10).

Thus, when there is a setting reception request, the setting values stored in the recorder 19 are read (S11) and are sent to the biological sample measuring devices 3 and 4 that requested them (S12).

Next, the biological sample measuring devices 3 and 4 receive the changed setting values for the biological sample measuring device 2 from the biological sample data management device 1 (S 13), and store them in their own operation setting memory 13 (S 14).

As discussed above, when the operation setting values of the biological sample measuring devices 2, 3, and 4 are changed, just the setting values of the biological sample measuring device 2 are changed with the input section 9, after which the setting values are received through communication with the biological sample data management device 1, allowing the operation setting values of the biological sample measuring devices 3 and 4 to be changed easily. As a result, a biological sample measurement system that is extremely convenient to use can be provided.

As discussed above, other patients besides the one being measured can be prevented from being awakened or bothered by beeping sounds by using the biological sample measuring devices 2, 3, and 4 for which the volume of the beeps has thus been lowered, to measure the blood glucose level of a patient after awakening.

### After Breakfast, etc.

Since it is assumed that all the patients will be awake during subsequent measurements taken before and after breakfast, before and after lunch, and before and after dinner, it is expected that the area around the measured patient will be fairly noisy. Thus, during these times, the setting is changed to increase the volume of the beeps so that the user can reliably confirm operation.

In view of this, with the biological sample measurement system in this embodiment, when the measurement of blood glucose level after awakening is finished, and the measurement value has been sent to the recorder 19 of the biological sample data management device 1, the input section 9 of the biological sample measuring device 2 is operated to change the setting values as to increase the volume of the beep again.

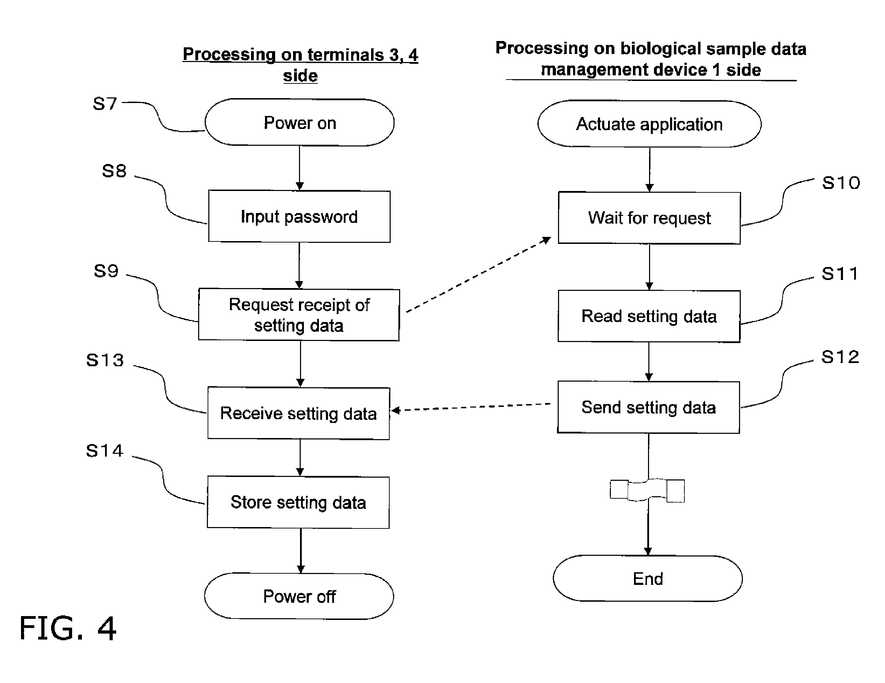

Again with the setting change here, the changed setting for the biological sample measuring device 2 is transmitted to the biological sample measuring devices 3 and 4 by the procedure shown in FIGS. 3 and 4 just as described above.

This allows the user to reliably confirm operation by hearing the beep emitted at a higher volume.

### Before Bedtime

When the measurement is made before the patient goes to sleep, consideration needs to be afforded, such as lowering the volume of the beep emitted from the operation sound generator 12 and decreasing the brightness of the display section 7, so as not to awaken any other patients who have already fallen asleep.

In view of this, in this embodiment after the measurement of blood glucose level after dinner has finished, and the measurement value has been sent to the recorder 19 of the biological sample data management device 1, the input section 9 of the biological sample measuring device 2 is operated to change the setting values so as to lower the volume of the beeping sound and decrease the brightness of the display section 7.

Again with the setting change here, the changed setting for the biological sample measuring device 2 is transmitted to the biological sample measuring devices 3 and 4 by the procedure shown in FIGS. 3 and 4 just as described above.

This allows the measurement to be taken in a state in which the beep is quiet and the brightness of the display section 7 is decreased. As a result, other patients who are sleeping are not awakened.

As discussed above, to measure biological samples in a hospital, the setting values of the biological sample measuring devices 2, 3, and 4 may need to be changed a number of times a day.

In view of this, with the biological sample measurement system in this embodiment, in actual practice the setting values for the biological sample measuring devices 3 and 4 are automatically changed merely by changing the setting values for the biological sample measuring device 2, which makes the system more convenient for the user to use.

Furthermore, in the above description an example was given in which the setting data for the biological sample measuring device 2 was changed and sent to the other biological sample measuring devices 3 and 4, but the change does not necessarily have to be based on the setting data for the biological sample measuring device 2. For instance, it may be based on the setting data for the biological sample measuring device (terminal) 3 or the biological sample measuring device (terminal) 4.

### Embodiment 2

The biological sample measurement system pertaining to another embodiment of the present invention will now be described.

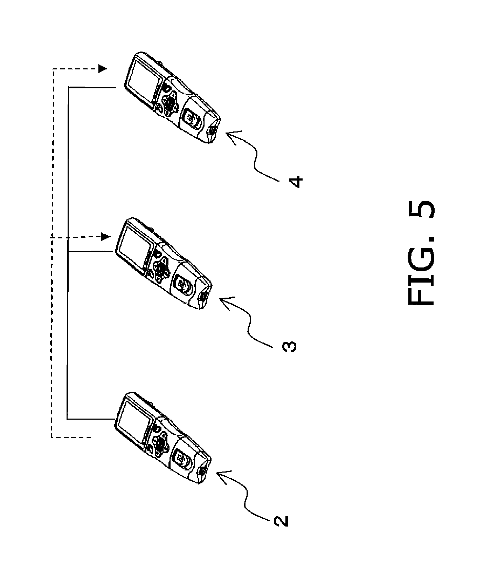

As shown in FIG. 5, the biological sample measurement system in this embodiment differs from that in Embodiment 1 above in that information about a change in the setting values is exchanged between the biological sample measuring devices 2, 3, and 4 without going through the biological sample data management device 1.

In this embodiment, those sections having the same function as in Embodiment 1 above are numbered the same and will not be described again.

Specifically, with the biological sample measurement system in this embodiment, as shown in FIG. 5, data linking related to setting changes is performed only by each of the biological sample measuring devices 2, 3, and 4, without going through the biological sample data management device 1.

This will be divided into two patterns and described.

### (i) Master Fixed

The biological sample measurement system of this embodiment can employ, for example, a method in which information related to various setting changes is exchanged between the biological sample measuring devices 2, 3, and 4 after the biological sample measuring device 2 has been fixed to a master terminal.

Specifically, the biological sample measuring devices 2, 3, and 4 are each equipped with a data transceiver 14 having a wireless or wired communication function.

Wired communication means include USB, LAN, RS-232C, and so on. Wireless communication means include wireless LAN, Bluetooth, infrared communication methods, and so on.

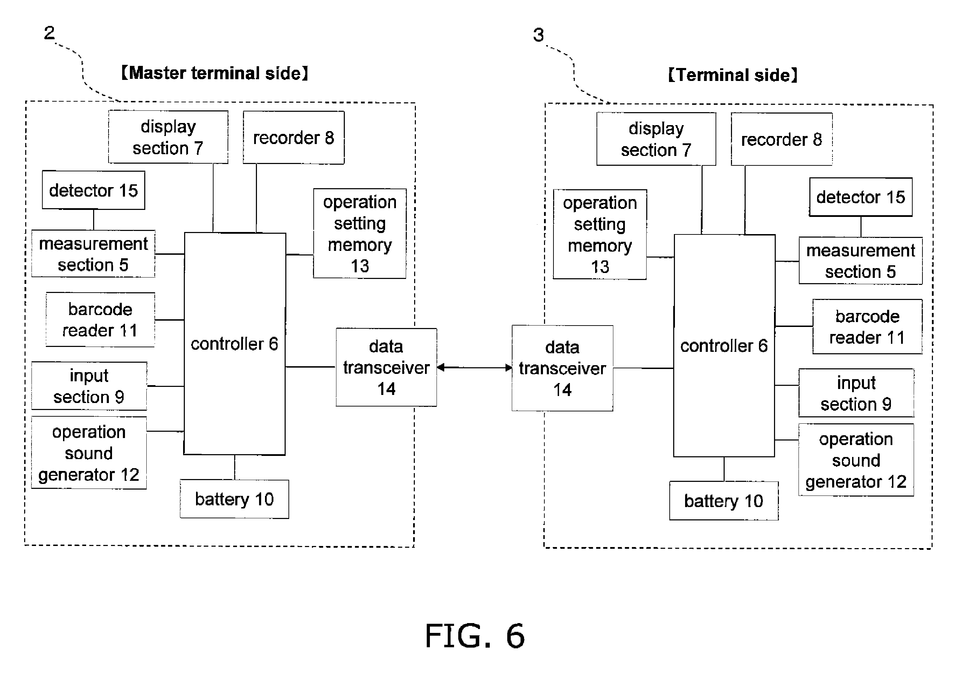

As shown in FIG. 6, in this case setting values are exchanged between the biological sample measuring device 2 on the master side and the biological sample measuring device 3 via the data transceiver 14.

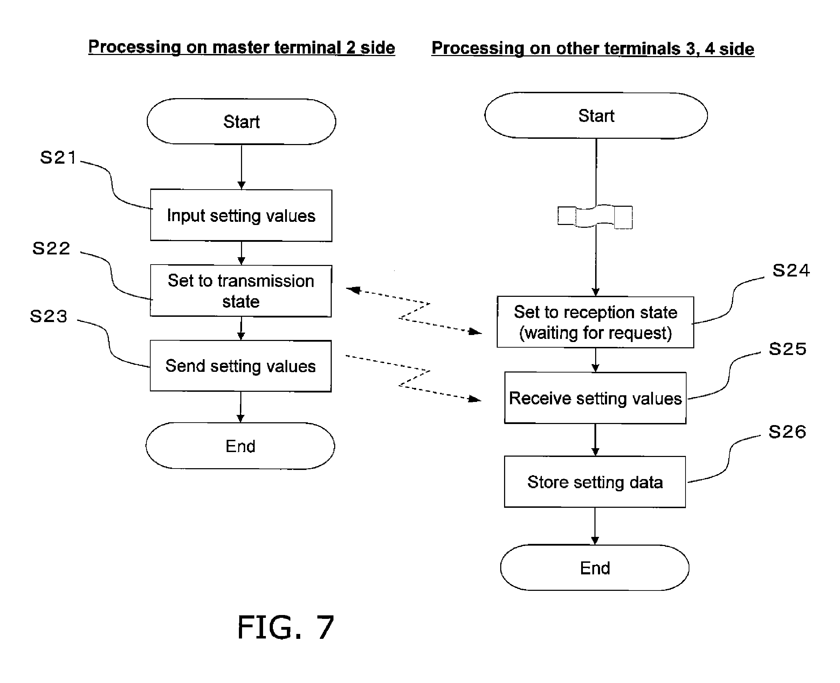

More specifically, as shown in FIG. 7, first the biological sample measuring device 2 on the master side inputs (changes) the beep volume, the brightness of the display section 7, and other such setting values as needed (S21).

Then, the biological sample measuring device 2 on the master side is put in a transmission state (S22).

Next, information related to setting values is sent from the biological sample measuring device 2 on the master side to the other biological sample measuring devices 3 and 4.

Meanwhile, on the other terminal side (the biological sample measuring devices 3 and 4), these are put in a reception state (S24), and information related to setting values is received from the biological sample measuring device 2 on the master side (S25).

Next, at the biological sample measuring devices 3 and 4, data for the setting values of the operation setting memory 13 inside each of the biological sample measuring devices 3 and 4 and so forth are updated and stored (S26).

The same applies when a biological sample measuring device is newly installed or replaced.

Consequently, setting data can be shared between the biological sample measuring device 2 on the master side and the other biological sample measuring devices 3 and 4, and setting values that are shared with the biological sample measuring device 2 can also be set at the biological sample measuring devices 3 and 4 merely by manually setting them at the biological sample measuring device 2 on the master side.

### (ii) Master Random

The biological sample measurement system in this embodiment can employ, for example, a method in which a biological sample measuring device in which setting data was initially changed is used as a master terminal, rather than fixing the biological sample measuring device that serves as the master terminal.

Specifically, in this case, in the same time slot, a biological sample measuring device in which setting values were initially changed is recognized as the master device, while other biological sample measuring devices are recognized as slave devices.

More specifically, when the setting data is changed for any one of the biological sample measuring devices (such as the biological sample measuring device 3), this is accompanied by the biological sample measuring device 3 entering into setting data transmission preparation as the master terminal. The processing in this case is such that it is received that the setting data has changed, a "master terminal" flag indicating that the device is the master terminal is switched on, and this device moves to a transmission state and enters into transmission preparation.

Next, when the other biological sample measuring devices 2 and 4 are connected for communication with the biological sample measuring device 3 on the master side, they enter into preparation for reception from the biological sample measuring device 3 on the master side. Consequently, they move to a state of waiting for a "transmission request" from the biological sample measuring device 3 on the master side.

Next, the biological sample measuring device 3 on the master side confirms that the biological sample measuring devices 2 and 4 are in a reception state (transmits a transmission request and confirms the reply to it), after which setting data and so forth are sent to the other biological sample measuring devices 2 and 4.

Next, the other biological sample measuring devices 2 and 4 receive setting data and so forth from the biological sample measuring device 3 on the master side, and this data is held and updated in the operation setting memory 13 installed internally in each of the biological sample measuring devices 2 and 4.

Consequently, just as with the above-mentioned master fixed method, in a biological sample measurement system including a plurality of biological sample measuring devices 2, 3, and 4, setting values that are shared with the biological sample measuring device 2 can also be set at the biological sample measuring devices 3 and 4 merely by manually setting them at the biological sample measuring device 2 on the master side.

Also, since the master device is not fixed, the biological sample measurement system can cope with a variety of situations, even if the biological sample measuring device serving as the master device malfunctions or is lost.

### Embodiment 3

The biological sample measurement system pertaining to yet another embodiment of the present invention will now be described.

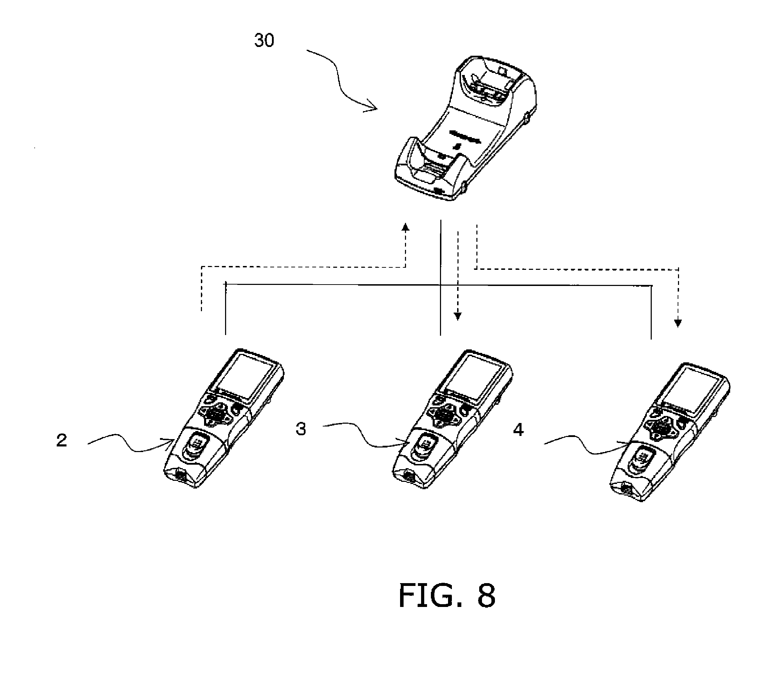

As shown in FIG. 8, the biological sample measurement system in this embodiment differs from that in Embodiment 1 above in that change information about setting values is exchanged between the biological sample measuring devices 2, 3, and 4 via a cradle 30 rather than the biological sample data management device 1.

In this embodiment, those sections having the same function as in Embodiment 1 above are numbered the same and will not be described again.

Specifically, with the biological sample measurement system in this embodiment, as shown in FIG. 8, change information about setting values is shared among the biological sample measuring devices 2, 3, and 4 via a cradle 30 provided for charging the biological sample measuring devices 2, 3, and 4.

More specifically, with the biological sample measurement system in this embodiment, first, setting data or the like for a specific terminal (the biological sample measuring device 2) is sent to the cradle 30.

The transmission of the setting data here can be accomplished by three methods: a wireless method, a wired method, or a method involving direct communication via a connector when the biological sample measuring device 2 is placed on the cradle 30.

Also, the following three possibilities exist for the timing at which the cradle 30 receives the setting data or the like of the specific biological sample measuring device 2.
- A method in which the specific biological sample measuring device 2 is actuated by pressing a control button (in this case, the biological sample measuring device 2 may or may not be placed on the cradle 30)
- A method in which setting data or the like about the terminal installed in the cradle 30 is automatically stored in a memory at the point when the biological sample measuring device 2 is placed on the cradle 30
- A method in which a "transmission and reception button" is provided to the cradle 30, and this "transmission and reception button" is pressed to receive setting data or the like for a specific terminal (the biological sample measuring device 2) at the cradle 30, and store this in an internal memory

Furthermore, the following three possibilities exist for the transmission timing at which the setting data held in the memory of the cradle 30 is sent to the other biological sample measuring devices 3 and 4.
- A method in which the specific biological sample measuring devices 3 and 4 are actuated by pressing control buttons (in this case, the biological sample measuring devices 3 and 4 may or may not be placed on the cradle 30)
- A method in which setting data or the like that has been automatically stored in the memory of the cradle 30 at the point when the biological sample measuring devices 3 and 4 are placed on the cradle 30 is sent to the biological sample measuring devices 3 and 4
- A method in which a "transmission and reception button" is provided to the cradle 30, and this "transmission and reception button" is pressed to send setting data or the like in the memory of the cradle 30 to the biological sample measuring devices 3 and 4 (the "transmission and reception button" provided to the cradle 30 may be separated into a "transmission button" and a "reception button")

On the cradle 30 side, which of the terminals is connected for communication (the biological sample measuring devices 2, 3, and 4) may be determined by using a "terminal ID" /"terminal number" or the like that is an identification code unique to each terminal.

For the specific terminal (biological sample measuring device), either a fixed method or random method may be employed, as in Embodiment 2 above.

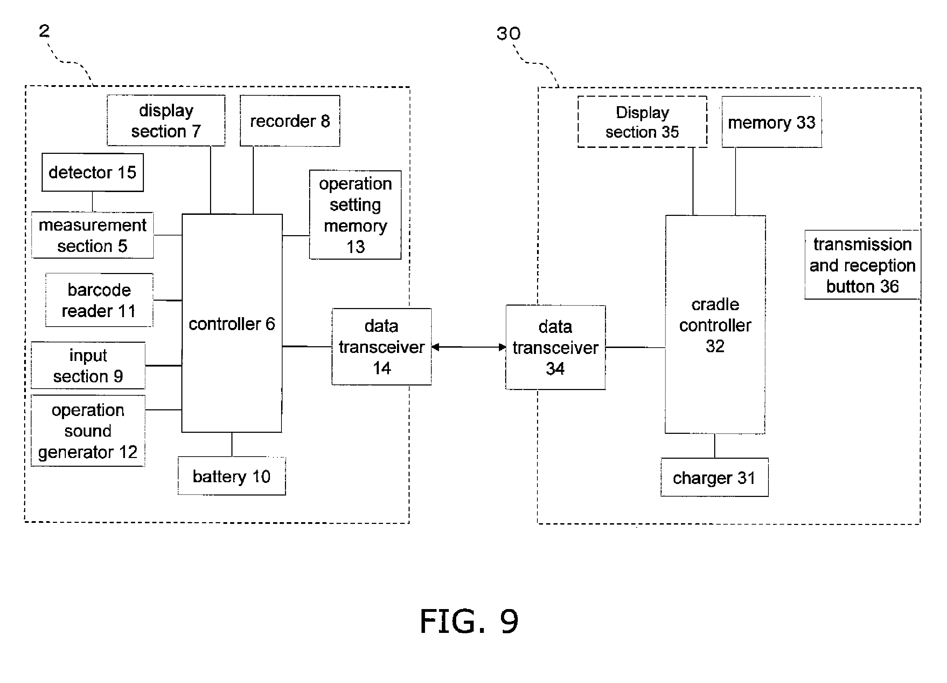

As shown in FIG. 9, the cradle 30 included in the biological sample measurement system of this embodiment has a charger 31, a cradle controller 32, a memory 33, a data transceiver 34, a display section 35, and a transmission and reception button 36.

The charger 31 charges the battery 10 installed in the biological sample measuring devices 2, 3, and 4 when the biological sample measuring device is placed on the cradle 30.

The cradle controller 32 controls the cradle 30 side, controls charging, sends and receives setting data, scans input sections such as the transmission and reception button 36, records and reproduces date to and from the memory 33, issues commands to display on the display section 35, and so forth.

The memory 33 holds setting data and so forth.

The data transceiver 34 sends and receives setting data and so forth between the data transceivers 14 of the biological sample measuring devices 2, 3, and 4.

The display section 35 may have an LCD (liquid crystal display) or other such display. The display section 35 displays the charge state, communication state, whether setting data exists and has been updated, and so on. The charge state, communication state, and setting data update state are displayed by lighting or flashing LED's, for example.

The transmission and reception button 36 is used in actuating data communication between the cradle 30 and the biological sample measuring devices 2, 3, and 4.

### Changing Setting values of Biological Sample Measuring Devices 2, 3, and 4

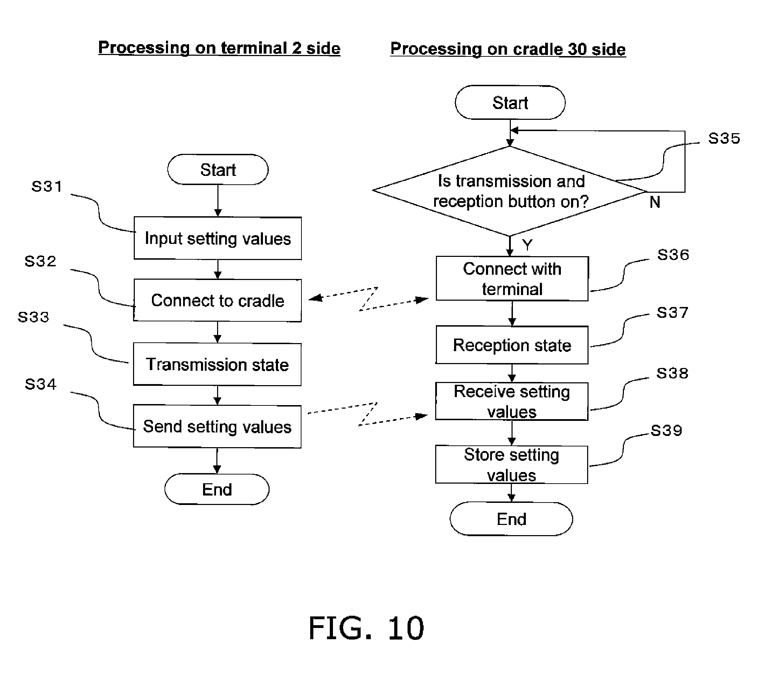

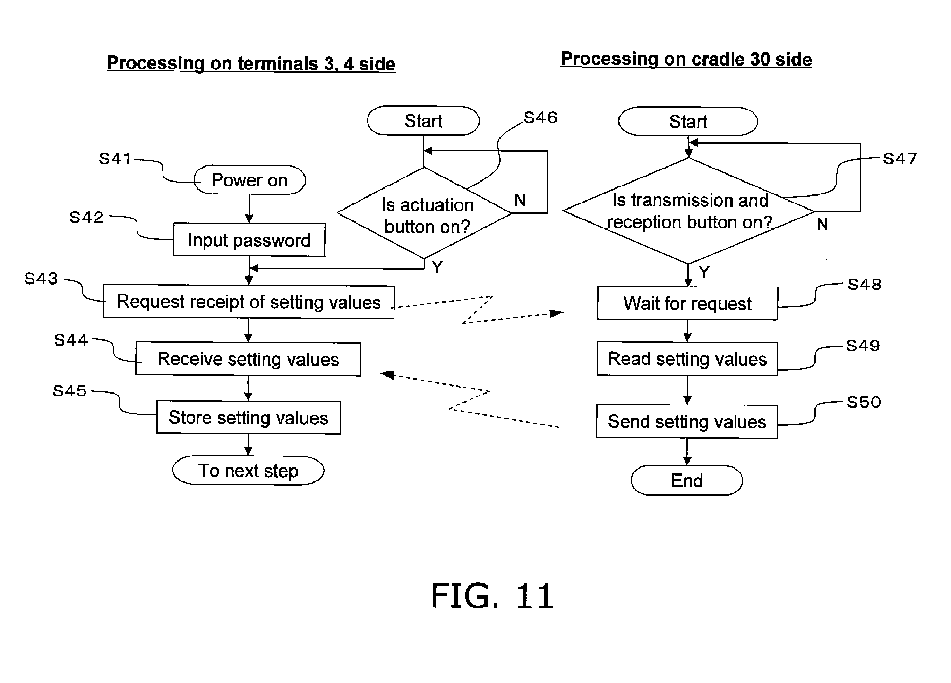

With the biological sample measurement system in this embodiment, sharing of the biological sample measuring devices 2, 3, and 4 is performed according to the procedure shown in FIGS. 10 and 11.

Specifically, FIG. 10 is a flowchart showing the flow up until the setting data for the biological sample measuring device 2 is sent to the cradle 30 and stored in a method in which actuation entails pressing the transmission and reception button 36 of the cradle 30.

First, the input and change of the setting values are performed through the input section 9 at the biological sample measuring device 2, for which the setting values have been changed manually by the user (S31).

Next, the biological sample measuring device 2 is connected for communication with the cradle 30 (S32).

In this case, the biological sample measuring device 2 does not necessarily need to be placed o the cradle 30. Also, the biological sample measuring device 2 side may be acutated by a method in which an actuation button of the input section 9 is pressed, or a method in which connection for communication is accomplished by automatically transitioning to a transmission state upon conclusion of the setting input operation.

Next, the biological sample measuring device 2 enters a state in which it can communicate with the cradle 30, and enters a state of transmission to the cradle 30 (S33).

Next, data related to the changed setting values is sent from the biological sample measuring device 2 to the cradle 30 (S34).

Meanwhile, on the cradle 30 side, the system waits until the transmission and reception button 36 (or reception button 36a) is pressed, and when it is pressed (S35), the cradle 30 is connected to the biological sample measuring device 2 (S36).

Next, the cradle 30 enters a state in which it can be connected for communication with the biological sample measuring device 2, and receives information related to setting values from the biological sample measuring device 2 (S38).

Next, the cradle 30 stores information related to setting values and received from the biological sample measuring device 2 in the internal memory 33 (S39).

FIG. 11 is a flowchart showing the flow up until the information related to changed setting values transmitted from the cradle 30, which received information related to setting values from the biological sample measuring device 2, is received by and stored in the other terminals (the biological sample measuring devices 3 and 4).

We will now describe a case in which information related to setting values is automatically requested when the power is turned on to the biological sample measuring devices 3 and 4.

First, when the power is turned on to the biological sample measuring devices 3 and 4 (S41), a password is inputted (S42). Instead of inputting a password, an ID or the like may be read by the barcode reader 11.

Next, the biological sample measuring devices 3 and 4 send the cradle 30 a request to receive information related to changed setting values (S43).

Next, the biological sample measuring devices 3 and 4 enter a state in which they can be connected for communication with the cradle 30, and receive information related to changed setting values from the cradle 30 (S44).

Next, the biological sample measuring devices 3 and 4 store the received information related to setting values in the operation setting memory 13 (S45).

When the actuation button on the biological sample measuring devices 3 and 4 is turned on, the flow may proceed to step 43 (S46).

Next, on the cradle 30 side, the system waits until the transmission and reception button 36 (or a transmission button 36b) is pressed, and when it is actually pressed (S47), the system enters a state of waiting for a reception request from the biological sample measuring devices 3 and 4 (S48).

Next, when a reception request is received from the biological sample measuring devices 3 and 4, the changed setting values are read from the memory 33 (S49) and sent to the biological sample measuring devices 3 and 4 (S50).

With the biological sample measurement system of this embodiment, information related to changed setting values in the biological sample measuring device 2 is shared between the other biological sample measuring devices 3 and 4 via the cradle 30, which is used to charge the biological sample measuring devices 2, 3, and 4.

Consequently, just as above, with a biological sample measurement system including a plurality of biological sample measuring devices 2, 3, and 4, setting values that are shared by the biological sample measuring device 2 can also be set in the other biological sample measuring devices 3 and 4 merely by manually setting the biological sample measuring device 2 on the master side.

### Embodiment 4

The biological sample measurement system pertaining to yet another embodiment of the present invention will now be described.

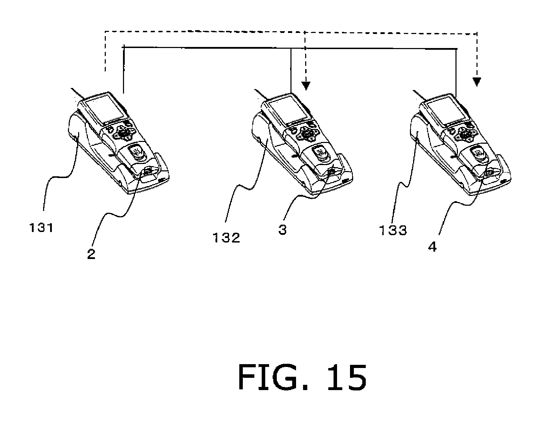

As shown in FIG. 15, the biological sample measurement system in this embodiment differs from that in Embodiment 1 above in that change information about setting values is exchanged between cradles 131, 132, and 133 on which the biological sample measuring devices 2, 3, and 4 are placed, respectively.

In this embodiment, those sections having the same function as in Embodiment 1 above are numbered the same and will not be described again.

Specifically, with the biological sample measurement system in this embodiment, communication is performed between the cradles 131, 132, and 133, updated setting data or the like held in the memory of the cradle serving as the master device is sent to the other cradles, and a corresponding terminal (biological sample measuring device) is set for each cradle.

In Embodiment 2 above, the transmission and reception of setting data were performed by a terminal (biological sample measuring device) alone, but this embodiment is different in that the transmission and reception of setting data are performed between the cradles 131, 132, and 133 in which the respective terminals (biological sample measuring devices 2, 3, and 4) are placed.

The cradles 131, 132, and 133 each comprise data transceivers 34A and 34B as communication sections having a wireless or wired communication function. In this case, wired methods include USB, LAN, RS-232C, and so on. Wireless methods include wireless LAN, Bluetooth, infrared communication methods, and so on.

In this embodiment, the cradles 131, 132, and 133 are provided corresponding to the respective terminals (biological sample measuring devices 2, 3, and 4).

Also, in this embodiment, just as in Embodiment 2 above, a specific cradle may be fixed as the master cradle of the system, or the master cradle may be selected at random.

In FIG. 15, a state is shown in which the biological sample measuring devices 2, 3, and 4 are respectively placed on the cradles 131, 132, and 133, but all of the biological sample measuring devices 2, 3, and 4 do not necessarily have to be placed on the cradles 131, 132, and 133. Thus, the setting values can be updated by placing the biological sample measuring devices 2, 3, and 4 in their corresponding cradles 131, 132, and 133 as needed.

Furthermore, in this embodiment, either the setting values must be entered from the biological sample measuring devices 2, 3, and 4 to the cradles 131, 132, and 133 ahead of time, or the biological sample measuring devices 2, 3, and 4 placed in the cradles 131, 132, and 133 during setting transmission or reception.

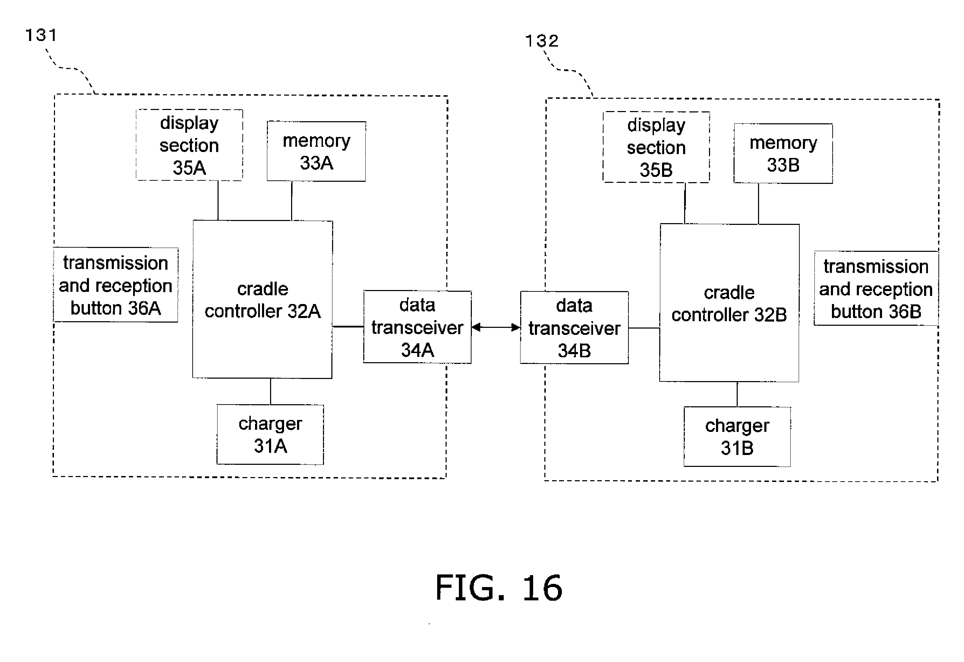

FIG. 16 is a block diagram of the configuration of the cradles 131 and 132 included in the biological sample measurement system of this embodiment.

The cradles 131 and 132 both have the same hardware configuration (the same applies to the cradle 133).

However, in this embodiment, since the setting values are exchanged between the cradles 131, 132, and 133, as shown in FIG. 16, this is performed via data transceivers 34A and 34B provided to the cradles 131 and 132, etc.

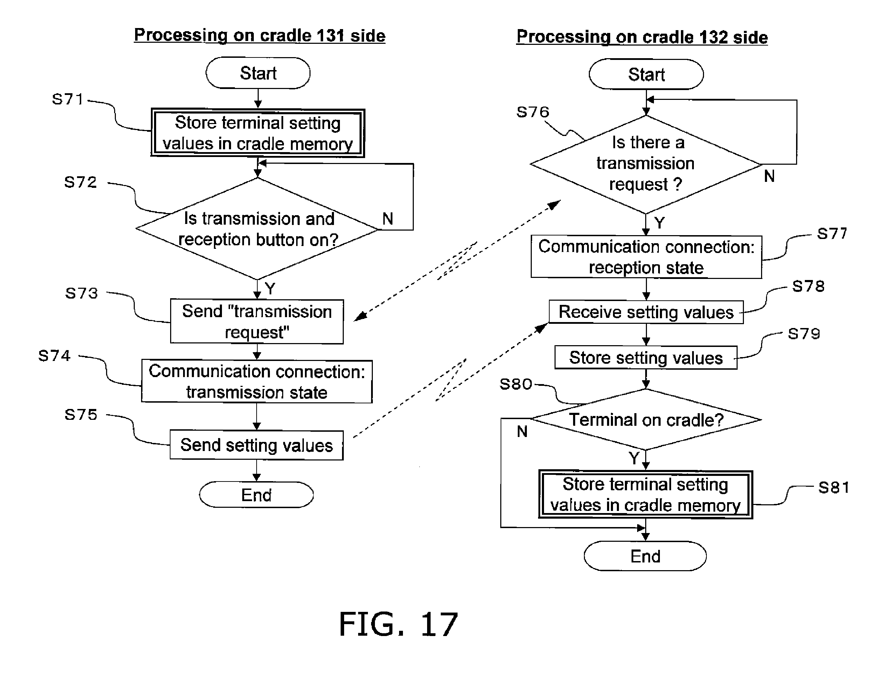

FIG. 17 is a flowchart of the procedure for changing setting values in the biological sample measuring devices 2, 3, and 4 of the biological sample measurement system in this embodiment.

We will now describe a case in which the setting values of a terminal (the biological sample measuring device 2) held in the cradle 131 (master cradle) of this embodiment are sent to the cradle 132.

The case here employs a method involving actuation by the transmission and reception buttons 36A and 36B of the cradle 131.

First, on the cradle 131 side, the setting values of the biological sample measuring device 2 are stored in a memory 33A of the cradle 131 (S71) (see the flowchart of Embodiment 3 (FIG. 10) for the method of putting the setting values into the memory 33A).

Next, when the reception button 36a is pressed (S72), a transmission request is sent to the cradle 132 (S73).

When the biological sample measuring device 2 has been placed in the cradle 131, a control button on the biological sample measuring device 2 may be pressed instead of pressing the reception button 36a of the cradle 131.

Next, the communication connection between the cradle 131 and the cradle 132 is checked (S74).

Once the communication connection is confirmed, information related to setting values is sent from the cradle 131 to the cradle 132 (S75).

Meanwhile, on the cradle 132 side, when it is confirmed that there is a "transmission request" from the cradle 131 (S76), a communication connection is established, and a reception state is entered (S77).

Next, the cradle 132 receives information related to setting values from the cradle 131 (S78).

Next, at the cradle 132, the received information related to setting values is stored in an internal memory 33B (S79).

Next, it is confirmed whether or not the biological sample measuring device 3 has been placed in the cradle 132 (S80), and if it has not, termination processing is performed (S80).

On the other hand, if the biological sample measuring device 3 has been placed in the cradle 132, information related to setting values is sent from the cradle 132 to the biological sample measuring device 3, the setting values are stored in the operation setting memory 13 of the biological sample measuring device 3 (S81), and the processing is ended (see the flowchart of Embodiment 3 (FIG. 11) for the method of sending the setting values of the memory 33B to the biological sample measuring device 3).

### Other Embodiments

Embodiments of the present invention were described above, but the present invention is not limited to or by the above embodiments, and various modifications are possible without departing from the gist of the invention.

### (A)

In the above embodiment, an example was given in which the setting values of the biological sample measuring device 2 were changed manually, and the setting values of the other biological sample measuring devices 3 and 4 were changed automatically, but the present invention is not limited to this.

For example, the setting values of the other biological sample measuring devices may be automatically changed using as a reference the biological sample measuring device whose setting values were changed initially, and any biological sample measuring device can serve as the master device that is the reference.

Also, the number of biological sample measuring devices included in the biological sample measurement system of the present invention is not limited to the three discussed above, and may be any number of two or more.

### (B)

In the above embodiment, an example was given in which the volume of beeps and the brightness of the display section 7 were used as the parameters whose setting was changed in the biological sample measuring devices 2, 3, and 4, but the present invention is not limited to this.

For example, the types of the above-mentioned beep include a sound indicating the completion of measurement, a warning sound indicating a measurement error, an alarm sound indicating the time when pre-scheduled measurement is to be carried out, and so forth, but of course these are not the only possibilities, and a melody sound may be used, or voice notification may be used.

Also, in addition to setting data pertaining to control of the beep sound, melody sound, or voice and of the brightness of the display section, the present invention can be similarly applied to cases in which other parameter setting values are changed, such as in changing and registering patient ID (identification) data accompanying a change in patient, or selecting approval (whether or not to input) for patient ID input setting; changing and registering nurse ID (identification) data accompanying a change in the nurse in charge (the user), or selecting approval (whether or not to input) for nurse ID input setting; changing measurement scheduling (shared portions); and so forth.

For example, in a hospital, it is necessary to carried out blood glucose measurements for many patients at once (nearly simultaneously) after they wake up.

That is, during this time slot many nurses are using many blood glucose measurement devices. Also, to manage nurse ID and patient ID at the same time, the input setting for nurse ID and patient ID is selected as "input," and this ID information is acquired simultaneously before measurement and used for accurate management.

However, during other times of the day, many blood glucose measurement devices are not necessarily being used all at once, so there are times when just some of the blood glucose measurement devices are used to measure outpatients or emergency patients. Since there is no need to input and manage various ID's in such cases, the setting is selected "do not input."

To accommodate situations such as these, it may at times be necessary to select and change input setting values for patient ID or nurse ID during the day, and the biological sample measuring device of the present invention can be used to advantage in such cases. This lightens the load on the nurses, and also eliminates problems such as setting errors, so reliability is enhanced.

### (C)

In the above Embodiment 3, an example was given in which data was exchanged between the biological sample measuring device 2 and the cradle 30 via wireless or wired communication means, but the present invention is not limited to this.

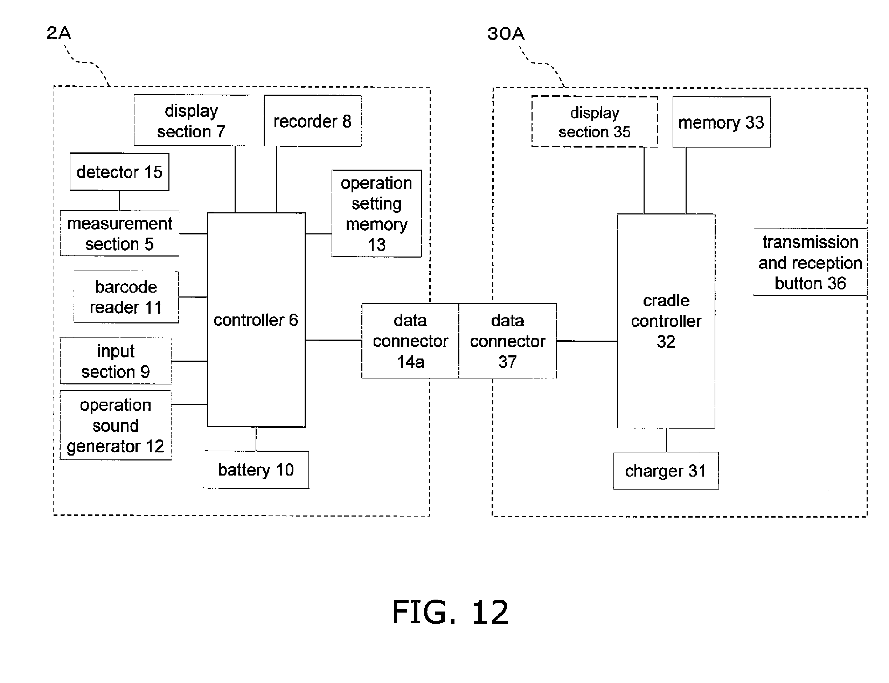

For example, as shown in FIG. 12, the biological sample measurement system may be such that data connectors 14a and 37 are provided instead of the data transceivers 14 and 34 as means for exchanging data between the biological sample measuring device 2 and the cradle 30, and data is exchanged between a biological sample measuring device 2A and a cradle 30A via the data connectors 14a and 37. The other biological sample measuring devices 3A and 4A have the same configuration as the biological sample measuring device 2A. The data connectors 14a and 37 shall be included as a type of data transceiver of the present invention.

In this case, when the biological sample measuring device 2A is placed on the cradle 30A, this electrically connects the data connector 14a and the data connector 37, allowing for the exchange of setting data and other such data.

With this configuration, setting data can be automatically sent back and forth between the biological sample measuring device 2A and the cradle 30A by using the data connector 37 to detect that the biological sample measuring device 2A has been placed on the cradle 30A. Thus, the "transmission and reception button 36" described in Embodiment 3 above is not necessarily needed.

The rest of the sections are the same as those in Embodiment 3 above.

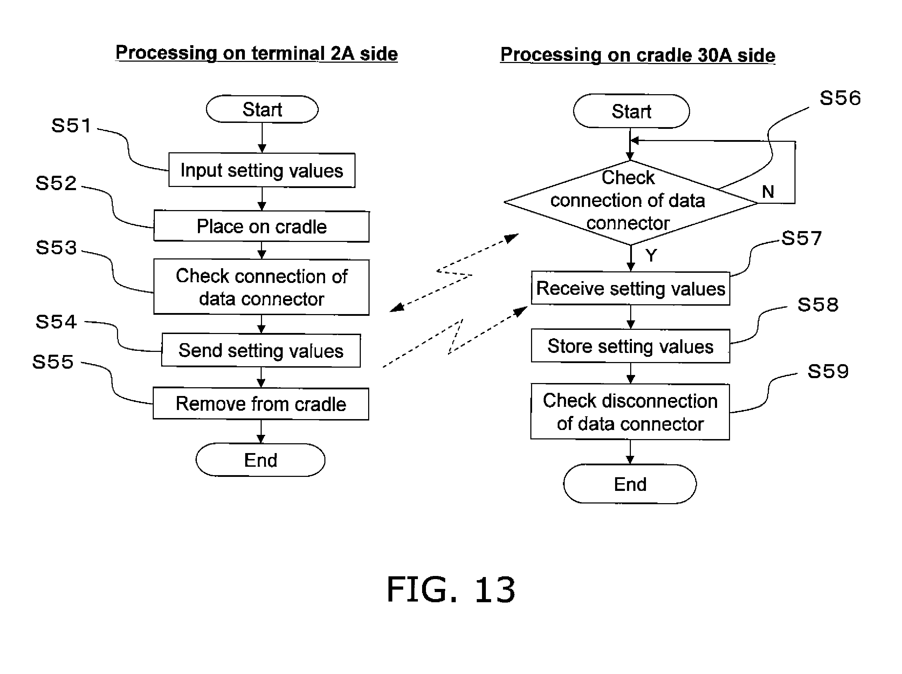

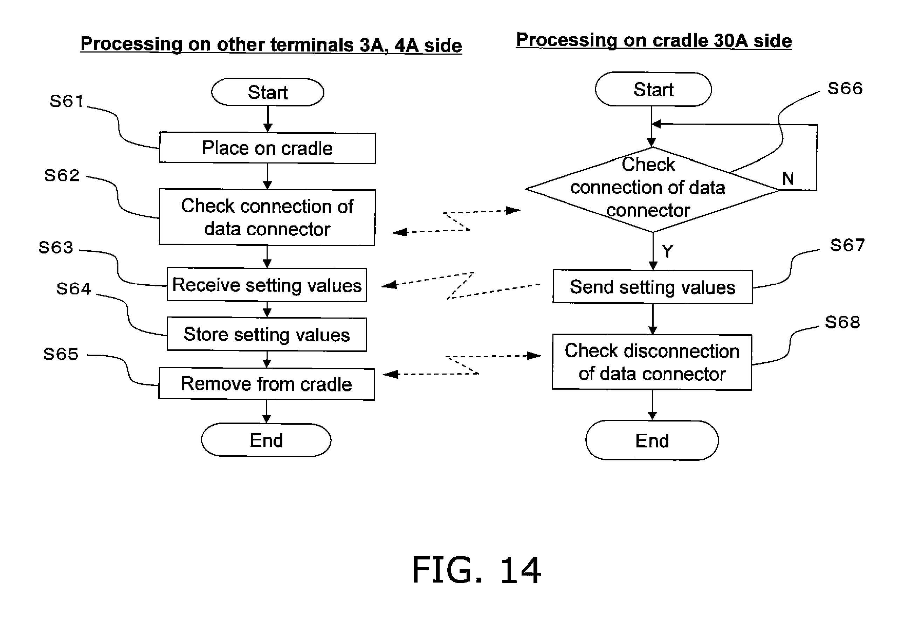

FIGS. 13 and 14 are flowcharts of the procedure for changing the setting values of the biological sample measuring devices in the biological sample measurement system shown in FIG. 12.

The flow shown here is for when employing a method in which actuation is automatically triggered by placing the biological sample measuring device 2A on the cradle 30A.

As shown in FIG. 13, with the biological sample measuring device 2A, first the setting values are inputted and changed via the input section 9 (S51).

Next, when the biological sample measuring device 2A is placed on the cradle 30A (S52), this completes the connection between the data connector 37 of the cradle 30A and the data connector 14a of the biological sample measuring device 2A (S53).

Next, the setting values of the biological sample measuring device 2A are sent to the cradle 30A (S54).

Next, the biological sample measuring device 2A is removed from the cradle 30A (S55), and processing is ended.

Meanwhile, on the cradle 30A side, first, the connection at the data connector 37 is checked (the placement of the biological sample measuring device 2A is detected) (S56).

Next, the cradle 30A receives the setting values of the biological sample measuring device 2A (S57).

The cradle 30A then stores the received setting values in the internal memory 33 (S58).

Next, disconnection at the data connector 37 is checked (it is detected that the biological sample measuring device 2A has been removed from the cradle 30A) (S59), and processing is ended.

Furthermore, as shown in FIG. 14, with the terminals (the biological sample measuring devices 3A and 4A), when they are placed on the cradle 30A (S61), the connection of the data connector 14a is checked (S62).

Next, the biological sample measuring devices 3A and 4A receive information related to setting values via the data connector 14a (S63).

The biological sample measuring devices 3A and 4A then store the received information related to setting values in the operation setting memory 13 (S64).

Next, the biological sample measuring devices 3A and 4A are removed from the cradle 30A (S65), and processing is ended.

Meanwhile, on the cradle 30A side, the connection with the biological sample measuring devices 3A and 4A is checked at the data connector 37 (S66).

Next, the cradle 30A sends the biological sample measuring devices 3A and 4A the information related to setting values received from the biological sample measuring device 2A (S67).

The data connector 37 then confirms that the biological sample measuring devices 3A and 4A have been removed from the cradle 30A (S68), and processing is ended.

### INDUSTRIAL APPLICABILITY

The effect of the present invention is that the setting of one biological sample measuring device is set to a first setting, thereby transmitting the first setting to a second biological sample measuring device, and the second biological sample measuring device is also set to the first setting value, so the setting of a plurality of biological sample measuring devices does not have to be carried out individually, making the setting of a plurality of biological sample measuring devices more efficient. Therefore, the present invention is expected to find use as a biological sample measurement system including a plurality of biological sample measuring devices used in hospitals.

### REFERENCE SIGNS LIST

1 biological sample data management device
2, 2A biological sample measuring device (first biological sample measuring device)
3, 3A, 4, 4A biological sample measuring device (second biological sample measuring device)
5 measurement section
6 controller
7 display section
8 recorder
9 input section
10 battery
11 barcode reader
12 operation sound generator
13 operation setting memory (recorder)
14 data transceiver
14a data connector
15 detector
16 data transceiver
17 controller
18 display section
19 recorder
20 input section
30, 30A cradle
31 charger
32 cradle controller
33 memory
34 data transceiver
34A, 34B data transceiver
35 display section
36 transmission and reception button
36a reception button
36b transmission button
37 data connector
131, 132, 133 cradle

## Claims

1. A biological sample measurement system, comprising:
first and second biological sample measuring devices that measure biological samples,
the first and second biological sample measuring devices each have:
a measurement section that measures biological samples;
a controller that is connected to the measurement section;
a display section that is connected to the controller and displays setting values and measurement results at the measurement section;
a recorder that stores the setting values and the measurement results;
an input section for inputting the setting values; and
a data transceiver for sending and receiving information related to the setting values,
at the first biological sample measuring device, the controller transmits information related to setting values that have been updated through the input section to the second biological sample measuring device via the data transceiver, and
at the second biological sample measuring device, the controller updates the setting values on the basis of information related to setting values transmitted from the first biological sample measuring device.

2. The biological sample measurement system according to Claim 1,
wherein the setting values include the brightness of the display section.

3. The biological sample measurement system according to Claim 1,
further comprising an operation sound generator that generates a beep when the input section is operated,
wherein the setting values include the volume of the beep.

4. The biological sample measurement system according to any of Claims 1 to 3,
further comprising a biological sample data management device that sends and receives information related to the setting values to and from the data transceiver of the first and second biological sample measuring devices,
wherein information related to the setting values that is sent from the first biological sample measuring device to the second biological sample measuring device is sent and received via the biological sample data management device.

5. The biological sample measurement system according to any of Claims 1 to 3,
further comprising a cradle into which the first and second biological sample measuring devices are placed, and which charges the first and second biological sample measuring devices and has a transceiver for sending and receiving information related to the setting values to and from the first and second biological sample measuring devices,
wherein the cradle sends information related to the setting values from the first biological sample measuring device to the second biological sample measuring device via the transceiver.

6. The biological sample measurement system according to Claim 1,
wherein the recorder that stores the setting values is made up of an EEPROM (electrically erasable programmable read-only memory).

7. The biological sample measurement system according to Claim 5,
wherein the cradle has a memory that holds setting data.

8. The biological sample measurement system according to Claim 5,
wherein the cradle has a display section that displays the communication status, the charging status, and the setting update status.

9. The biological sample measurement system according to Claim 5,
wherein the cradle has a transmission and reception button that starts communication.

10. The biological sample measurement system according to Claim 5,
wherein the cradle has a data connector that sends and receives data directly to and from the biological sample measuring devices.

11. The biological sample measurement system according to Claim 5,
wherein the biological sample measuring devices have a data connector that sends and receives data directly to and from the cradle.

12. The biological sample measurement system according to Claim 1 or 5,
wherein the biological sample measuring devices have internal device identification codes.
